# EUROPÄISCHE PATENTANMELDUNG

(11) **EP 2 266 529 A2**
(43) Veröffentlichungstag der Anmeldung: **29.12.2010**
(21) Anmeldenummer: 10158583.4
(22) Anmeldetag: 31.03.2010
(51) Int. Cl.: A61K 8/49, A61K 8/66, A61K 8/97, A61Q 19/08

(54) **UV-Schutz-Kosmetikum**

(30) Priorität: 25.06.2009 DE 102009027199
(71) Anmelder: Henkel AG & Co. KGaA, 40589 Düsseldorf (DE)
(72) Erfinder: Holtkötter, Olaf, 50354, Hürth (DE); Bock, Andreas, 40591, Düsseldorf (DE); Janßen, Frank, 41470, Neuss (DE)

(57) **Zusammenfassung**

Die Wirkung von dedifferenzierten Pflanzenzellen in kosmetischen Produkten läßt sich durch den zusätzlichen Einsatz von 6,7-disubstituierten 2,2-Dialkylchromanen oder -chromenen signifikant steigern. Bevorzugte kosmetische oder dermatologische topische Zusammensetzungen, enthalten in einem geeigneten kosmetischen oder dermatologischen Träger mindestens eine Substanz, ausgewählt aus 6,7-disubstituierten 2,2-Dialkylchromanen oder -chromenen der allgemeinen Formeln (I) oder (II), 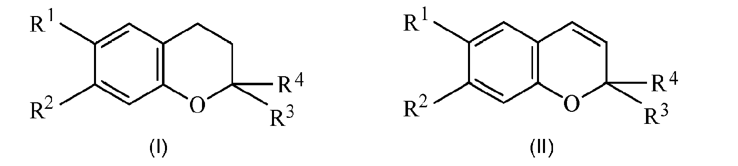 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6 und mindestens eine dedifferenzierte Pflanzenzellsuspension.

## Beschreibung

Die Erfindung betrifft topische kosmetische oder dermatologische Zusammensetzungen zur Hautbehandlung, die der Hautalterung und deren Anzeichen entgegenwirken.

Die Epidermis der Haut unterliegt einem ständigen Erneuerungsprozess, in dem Keratinozyten aus dem an der dermal-epidermalen Grenzschicht liegendem Stratum basale in Richtung Hautoberfläche wandern. Dabei differenzieren sie und bilden zunächst das Stratum spinosum, das Stratum granulosum und schließlich das Stratum corneum, die Hornschicht, in der die ehemals lebenden Keratinozyten nur noch als tote Schuppen zu finden sind, die durch mechanischen Stress an der Hautoberfläche kontinuierlich abgeschilfert werden. Die Quelle für die neuen Zellen im Stratum basale bilden die epidermalen Stammzellen, die somit für den Erhalt einer biologisch intakten Epidermis essentiell sind.

Die Alterung der Haut wird durch die sich im unterschiedlichem Maße überlagernden Prozesse der chronologischen (intrinsischen) Alterung und der extrinsischen Alterung bestimmt, wobei bei letzterer die durch UV-Strahlung verursachte Photoalterung den größten Anteil hat. UV-Strahlung erzeugt in den Zellen der Epidermis und der Dermis u.a. oxidativen Stress und DNA-Schäden, was zu einer verminderten Leistungsfähigkeit der Zellen und schließlich zum Zelltod führt. Histologisch äußert sich die Photoalterung der Haut in der Epidermis in Abhängigkeit vom Hauttyp in einer Hyperplasie, also einer deutlichen Verdickung der Epidermis (Hauttyp III-V), oder einer Atrophie, einer dünner werdenden Epidermis (Hauttyp I - II). Während ersteres ein gesteigertes Proliferations- und verändertes Differenzierungsverhalten voraussetzt, geht eine Verdünnung mit einer reduzierten Proliferationsrate und dem vorzeitigen Zelltod einher.

In humaner Haut wurden Studien zu UV-induzierten Schäden bislang ausschließlich an Gesamtpräparationen von Keratinozyten beschrieben, die immer eine undefinierte Mischung aus epidermalen Stammzellen und in verschiedenen Differenzierungsstadien befindlichen Keratinozyten darstellen.

Eigene Untersuchungen zeigen, dass epidermale Stammzellen durch UV-Strahlung stark geschädigt werden, was sich in einer reduzierten Vitalität, einer erhöhten Caspase 3/7-Aktivität und einer gesteigerten IL-8-Ausschüttung äußert. Der komplette Verlust der weiteren Teilungsfähigkeit weist auf eine stärkere Reaktion der epidermalen Stammzellen auf die Bestrahlung hin, als es für Gesamtpräparationen von Keratinozyten beschrieben ist, die eine verminderte Teilungsfähigkeit beibehalten.

In der EP 1 985 280 A2 wird die Verwendung von dedifferenzierten Pflanzenzellen in kosmetischen Produkten zum Schutz von Stammzellen gegen intrinsische und extrinsische Stressfaktoren, insbesondere zur Förderung der Proliferation von Stammzellen und deren Schutz vor Apoptose (Zelltod) beschrieben. Diese Schrift bezieht sich im Besonderen auf die Verwendung von dedifferenzierten Pflanzenzellen aus den Früchten von Malus domestica (Apfel) der Sorte Uttwiler Spätlauber (Malus domestica Cultivar Uttwiler Spätlauber).

Es wurde nun gefunden, dass sich die Wirkung von dedifferenzierten Pflanzenzellen in kosmetischen Produkten durch den zusätzlichen Einsatz von 6,7-disubstituierten 2,2-Dialkylchromanen oder-chromenen signifikant steigern läßt.

Gegenstand der vorliegenden Erfindung sind in einer ersten Ausführungsform kosmetische oder dermatologische topische Zusammensetzungen, enthaltend in einem geeigneten kosmetischen oder dermatologischen Träger
a) mindestens eine Substanz, ausgewählt aus 6,7-disubstituierten 2,2-Dialkylchromanen oder -chromenen der allgemeinen Formeln (I) oder (II), 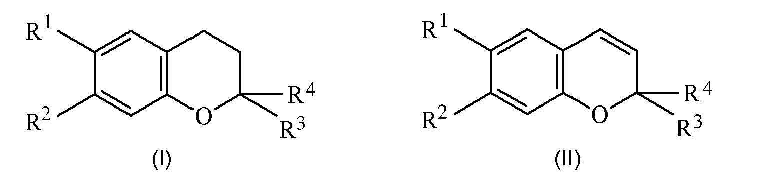 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6
b) mindestens eine dedifferenzierte Pflanzenzellsuspension.

Als Inhaltsstoff a) enthalten die erfindungsgemäßen Mittel 6,7-disubstituierte 2,2-Dialkylchromane oder -chromene der allgemeinen Formeln (I) oder (II), 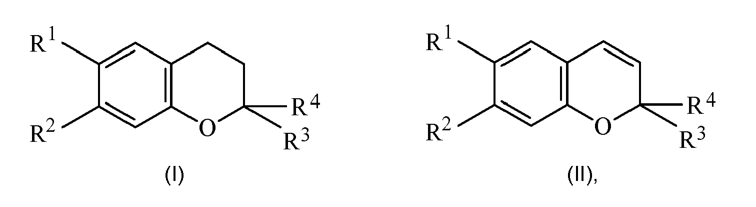 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen.

Die Substituenten R¹ und R² sind unabhängig voneinander ausgewählt aus einer OH-Gruppe, einer Methoxy-Gruppe und einer CF₃CH₂O-Gruppe. In einer bevorzugten Ausführungsform sind R¹ und R² unabhängig voneinander ausgewählt aus einer OH-Gruppe und einer Methoxy-Gruppe. In einer besonders bevorzugten Ausführungsform stellen R¹ eine OH-Gruppe und R² eine Methoxy-Gruppe dar.

Die Substituenten R³ und R⁴ stellen unabhängig voneinander eine C₁-C₄-Alkylgruppe dar. Unter C₁-C₄-Alkylgruppe ist dabei erfindungsgemäß eine Methyl-, Ethyl-, n-Propyl-, Isopropyl-, n-Butyl-, Isobutyl- beziehungsweise 2-Methylpropyl-, sec-Butyl- beziehungsweise 1-Methylpropyl- oder eine tert.-Butyl-Gruppe zu verstehen. In einer bevorzugten Ausführungsform sind R³ und R⁴ unabhängig voneinander ausgewählt aus einer Methyl-, Ethyl-, n-Propyl-, Isopropyl- und einer n-Butyl-Gruppe. Besonders bevorzugt stellen R³ und R⁴ unabhängig voneinander eine Methyl- oder Ethylgruppe dar. Außerordentlich bevorzugt ist, dass R³ und R⁴ identisch sind.

Erfindungsgemäß bevorzugt verwendet werden die 6,7-disubstituierten 2,2-Dialkylchromane. Ein erfindungsgemäß außerordentlich bevorzugt verwendeter Wirkstoff ist 2,2-Dimethyl-6-hydroxy-7-methoxy-chroman mit der systematischen Bezeichnung 3,4-Dihydro-7-methoxy-2,2-dimethyl-2H-1-benzopyran-6-ol und der INCI-Bezeichnung Dimethylmethoxy Chromanol. Die Substanz ist unter dem Handelsnamen Lipochroman-6 von der Firma Lipotec S.A. erhältlich. Die 6,7-disubstituierten 2,2-Dialkylchromane oder -chromene der allgemeinen Formeln (I) oder (II) werden vorzugsweise in Mengen von 0,001 - 5 Gew.-%, bevorzugt 0,005 - 2 Gew.-% und besonders bevorzugt 0,01 - 1 Gew.-%, jeweils bezogen auf die gesamte Zusammensetzung, eingesetzt. Zusammenfassend sind erfindungsgemäße Zusammensetzungen bevorzugt, die - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (II) oder (III) wie vorstehend definiert, enthalten, wobei bevorzugte Mittel - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten. Weitere erfindungsgemäß bevorzugte Zusammensetzungen enthalten - bezogen auf ihr Gewicht - eine Mischung aus der oxidierten und der reduzierten Form von 3,4-Dihydro-7-methoxy-2,2-dimethyl-2H-1-benzopyran-6-ol in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%.

Als Inhaltsstoff b) enthalten die erfindungsgemäßen Mittel mindestens eine dedifferenzierte Pflanzenzellsuspension. Verfahren der Pflanzen-Zellkultur-Technik beinhalten Techniken, die es ermöglichen unter Einhaltung gewisser bekannter Verfahrensschritte uniforme dedifferenzierte Zellen zu erhalten, welche folgende Vorteile gegenüber ganzen kultivierten Pflanzen aufweisen: Unabhängigkeit von Jahreszeiten; kontinuierliche Produktion; frei von Umweltverschmutzungen und anderen Unreinheiten; kontrollierbare und reproduzierbare Produktion von Metaboliten im Bezug auf Quantität und Qualität; Schutz seltener oder beschränkter Pflanzenreserven; keine Einschränkung der Marktverfügbarkeit.

Die Grundlage der Kultivierung solcher dedifferenzierter Pflanzenzellen nützt den biologischen Umstand, dass jede Pflanzenzelle die Fähigkeit in sich trägt, die ganze Pflanze, von der sie herstammt zu bilden. Diese Fähigkeit nennt sich Totipotenz und ist vergleichbar mit der Pluripotenz von animalen embryonalen Stammzellen. Deshalb ist anzunehmen, dass dedifferenzierte Pflanzenzellen einen positiven Einfluss haben auf den Schutz und die Aktivierung von Hautstammzellen.

Die Herstellung geeigneter Extrakte umfasst bevorzugt die folgenden Hauptschritte:
a) Erstellung einer stabilen dedifferenzierten Zelllinie im Labormaßstab;
b) Massenkultivierung der Zellen in einem Einwegbeutel-Reaktorsystem (sog. Wave-Reaktor); und
c) Herstellung eines Total-Extraktes mittels Hochdruckhomogenisation unter Verwendung von leeren Liposomen.

Zweckmäßigerweise folgt man dabei beim Schritt c) dem folgenden Verfahrensablauf: Aufschluss der Pflanzenzellen durch Hochdruckhomogenisation; Extraktion und Stabilisierung von Inhaltsstoffen mit Liposomen; wobei beide Teile des Verfahrensablaufs in einem einzigen Verfahrensschritt gleichzeitig durchgeführt werden. Detaillierte Beschreibung der Erfindung: Induktion und Stabilisierung der Zelllinie (a)

Folgende Schritte führen zu einer dedifferenzierten Zelllinie aus pflanzlichem Gewebe:
(a1) Auswahl eines geeigneten Gewebes für die Induktion;
(a2) Oberflächensterilisation;
(a3) Plattieren der Explantate auf einem geeigneten Festmedium zur Kallusinduktion;
(a4) Ernte des Kallus, der auf der verletzten Oberfläche der Explantate gewachsen ist;
(a5) Subkultivierung des so gewonnenen Kallus auf demselben Medium bis die Zellen voll dedifferenziert sind;
(a6) Einbringen der dedifferenzierten Zellen in ein geeignetes Flüssigmedium;
(a7) Homogenisation der Zellen in Suspension, bis keine großen Zellklumpen mehr vorhanden sind; und
(a8) Subkultivierung und kontinuierliche Charakterisierung der Zellsuspension.

Erfindungsgemäß besonders bevorzugte Zusammensetzungen sind **dadurch gekennzeichnet, dass** sie mindestens eine dedifferenzierte Pflanzenzellsuspension von Pflanzen der Unterfamilie Maloideae (Kernobstgewächse) enthalten.

Die Kernobstgewächse wurden lange als eine der vier Unterfamilien der Rosengewächse geführt. Molekulargenetische Untersuchungen zeigten, dass sie eine Gruppe innerhalb der Spiraeoideae bilden. Potter et al. haben die Gruppe daher als Subtribus Pyrinae definiert. Zusammen mit den Gattungen *Kageneckia, Vauquelinia* und *Lindleya* bilden sie die Tribus Pyreae.

Die Pflanzensippe Malodieae umfaßt beispielsweise die Pflanzengattungen
- Felsenbirnen (*Amelanchier*)
- *Aria*
- Apfelbeeren (*Aronia*)
- Zierquitten (*Chaenomeles*)
- *Chamaemeles*
- *Chamaemespilus*
- *Cormus*
- Zwergmispeln (*Cotoneaster*)
- Weißdorne (*Crataegus*)
- Quitten (*Cydonia*)
- *Dichotomanthes*
- *Docynia*
- *Docyniopsis*
- Wollmispeln (*Eriobotrya*)
- *Eriolobus*
- *Hesperomeles*
- *Heteromeles*
- Äpfel (*Malus*)
- *Melacomenes*
- Mispeln (*Mespilus*)
- *Osteomeles*
- *Peraphyllum*
- Glanzmispeln (*Photinia*)
- *Pseudocydonia*
- Feuerdorn (*Pyracantha*)
- Birnen (*Pyrus*)
- *Rhaphiolepis*
- Mehlbeeren (*Sorbus*)
- *Stranvaesia*
- *Torminalis*

Innerhalb dieser Gattungen sind einige erfindungsgemäß besonders geeignet. Besonders bevorzugt werden Pflanzenzellsuspension von Pflanzen der Gattung Pyrus (Birnen) und Malus (Äpfel) erfindungsgemäß eingesetzt.

Besonders bevorzugte erfindungsgemäße Zusammensetzungen sind **dadurch gekennzeichnet, dass** sie mindestens eine dedifferenzierte Pflanzenzellsuspension von Pflanzen der Gattung Malus (Äpfel) enthalten, wobei bevorzugte Pflanzenzellen ausgewählt sind aus Zellen von
■ *alus aldenhamensis*
■ *Malus angustifolia*
■ *Malus* × *arnoldiana* (*M. baccata* × *M. floribunda*)
■ *Malus asiatica*
■ *Malus* × *atrosanguineum* (*M. halliana* × *M. sieboldii*)
■ *Malus baccata*
■ *Malus bracteata*
■ *Malus brevipes*
■ *Malus coronaria*
■ *Malus daochengensis*
■ *Malus dasyphylia*
■ *Malus* × *domestica*
■ *Malus doumeri* (Bois) A.Chevalier
■ *Malus florentina*
■ *Malus floribunda*
■ *Malus formosana*
■ *Malus fusca*
■ *Malus glabrata*
■ *Malus glaucescens*
■ *Malus halliana*
■ *Malus hilleri*
■ *Malus honanensis*
■ *Malus hupehensis*
■ *Malus ioensis*
■ *Malus kansuensis*
■ *Malus komarovii*
■ *Malus lancifolia*
■ *Malus leiocalyca*
■ *Malus* × *magdeburgensis*
■ *Malus mandschurica*
■ *Malus melliana*
■ *Malus* × *micromalus* Makino
■ *Malus niedzwetskyana*
■ *Malus ombrophila*
■ *Malus platycarpa*
■ *Malus praecox*
■ *Malus prattii*
■ *Malus prunifolia*
■ *Malus pumila* Mill.
■ *Malus* × *purpurea*)*,* (*M. atrosanguinea* × *M. niedzwetskyana*)
■ *Malus rockii*
■ *Malus sargentii* (*Syn.: M. toringo* ssp. *sargentii*)
■ *Malus sieboldii* (*Syn.: M. toringo*)
■ *alus* × *scheideckeri*
■ *alus sikkimensis*
■ *Malus sieversii*
■ *Malus* × *soulardi*
■ *Malus spectabilis*
■ *Malus sublobata*
■ *Malus sylvestris*
■ *Malus toringo*
■ *Malus toringoides*
■ *Malus transitoria*
■ *Malus trilobata*
■ *Malus tschonoskii*
■ *Malus yunnanensis*

Als ganz besonders bevorzugt hat sich eine alte Apfelsorte erwiesen. Bevorzugte erfindungsgemäße Zusammensetzungen sind daher **dadurch gekennzeichnet, dass** sie mindestens eine dedifferenzierte Pflanzenzellsuspension von Pflanzen von Malus domestica Cultivar Uttwiler Spätlauber (Apfel Sorte Uttwiler Spätlauber) enthalten.

Unabhängig davon, ob die erfindungsgemäßen Zusammensetzungen eine oder mehrere Pflanzenzellsuspension(en) enthalten und unabhängig davon, welche Pflanzenzellsuspension(en) eingesetzt wird/werden, sind erfindungsgemäße Zusammensetzungen bevorzugt, die - bezogen auf ihr Gewicht - 0,001 bis 20 Gew.-%, vorzugsweise 0,002 bis 17,5 Gew.-%, weiter bevorzugt 0,005 bis 15 Gew.-%, noch weiter bevorzugt 0,01 bis 10 Gew.-% und insbesondere 0,05 bis 7 Gew.-% Pflanzenzellsuspension(en) enthalten.

Entsprechende Pflanzenzellsuspensionen sind kommerziell unter dem Namen PhytoCellTec von der Firma Mibelle Biochemistry erhältlich.

Durch die Verwendung von 6,7-disubstituierten 2,2-Dialkylchromanen oder -chromenen, insbesondere von Lipochroman-6 und Malus Domestica-Extrakt lassen sich epidermale Stammzellen vor UV-induzierten Schäden schützen, wodurch die Homöostase der Epidermis ungestört bleibt und photoalterungsbedingten Hautveränderungen entgegengewirkt wird.

Es hat sich gzeigt, dass bestimmte Inhaltsstoffe die positiven Wirkungen der erfindungsgemäßen Kombination weiter verstärken können.

Als eine weitere besonders geeignete Verbindung können die erfindungsgemäßen Mittel mindestens einen Stoff aus der Gruppe der Superoxiddismutasen enthalten. Erfindungsgemäß bevorzugte Zusammensetzungen enthalten- bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 2 Gew.-% Superoxiddismutase(n).

Weitere besonders bevorzugte erfindungsgemäße Zusammensetzungen enthalten - bezogen auf ihr Gewicht - 0,001 bis 10 Gew.-% mindestens eines Wirkstoffes aus der Gruppe
- der Monomeren, Oligomeren und Polymeren von Aminosäuren, N-C₂-C₂₄-Acylaminosäuren, den Estern und/oder den physiologisch verträglichen Metallsalzen dieser Substanzen und/oder
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- der Extrakte aus den Blüten von Lonicera japonica und/oder
- der Extrakte aus den Früchten von Xanthium sibiricum und/oder
- der Extrakte aus den Wurzeln von Cyperus rotundus und/oder
- Apfelkernextrakt und/oder
- Hyaluronsäure und/oder
- der Phytohormone und/oder
- der Isoflavonoide und/oder
- der Phytosterole und/oder
- der Triterpenoide und/oder
- der Tocopherole
- Ellagsäure und/oder Ellagate und/oder
- der Xanthophylle, insbesondere Astaxanthin, und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten.

Bevorzugte erfindungsgemäße Zusammensetzungen sind **dadurch gekennzeichnet, dass** die Monomeren von Aminosäuren, N-C₂-C₂₄-Acylaminosäuren und/oder den physiologisch verträglichen Metallsalzen dieser Substanzen ausgewählt sind aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.

Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β (*tissue growth factor*), der die Synthese von Collagen in dermalen Fibroblasten induziert, freizusetzen. Derartige Aminosäureoligomere können als Wirkstoffe gegen die Hautalterung verwendet werden.

Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter dem Namen "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produkts Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im Folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus Gin und den Aminosäuren, die Gin substituieren können, Xcc ausgewählt ist aus Pro oder Arg und den Aminosäuren, die Pro und Arg substituieren können. Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile). Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin. Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin. Als Rigin-Analoga sind erfindungsgemäß Gly-Gln-Arg-Pro und Val-Val-Arg-Pro bevorzugt. ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt. Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).

Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich.

Proteinhydrolysate können naturgemäß auch monomere Aminosäuren und Oligopeptide enthalten; ihre Zusammensetzung ist normalerweise nicht definiert.

Ebenfalls bevorzugt ist der Einsatz von Acylderivaten der Proteinhydrolysate, z. B. in Form ihrer Fettsäure-Kondensationsprodukte.

Zusammenfassend sind erfindungsgemäß bevorzugte Zusammensetzungen dadurch gekennzeichnet, dass die Oligomeren von Aminosäuren, N-C₂-C₂₄-Acylaminosäuren und/oder den physiologisch verträglichen Metallsalzen dieser Substanzen ausgewählt sind aus Di-, Tri-, Tetra-, Penta-, Hexa- oder Pentadecapeptiden, die N-acyliert und/oder verestert sein können.

Ganz besonders bevorzugte erfindungsgemäße Zusammensetzungen sind dadurch gekennzeichnet, dass das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

Ebenfalls bevorzugt sind Zusammensetzungen, bei denen die Polymeren von Aminosäuren, N-C₂-C₂₄-Acylaminosäuren und/oder den physiologisch verträglichen Metallsalzen dieser Substanzen ausgewählt sind aus tierischen und pflanzlichen Proteinhydrolysaten, tierischen und pflanzlichen Proteinen und DNA-Reparaturenzymen.

Hier sind ganz besonders bevorzugte erfindungsgemäße Zusammensetzungen dadurch gekennzeichnet, dass sie zusätzlich mindestens ein DNA-Reparaturenzym, vorzugsweise Photolyase und/oder T4 Endonuclease V und/oder 8-Oxoguanin-glycosylase und/oder deren Mischungen enthalten.

Die DNA-Reparaturenzyme können sowohl in Liposomen verkapselt als auch frei, das heißt unverkapselt, vorliegen. Die Liposomenverkapselung kann bevorzugt mit Phospholipiden, besonders bevorzugt mit Lecithin, erfolgen. Erfindungsgemäß besonders bevorzugt ist der Einsatz von mindestens einem liposomenverkapselten DNA-Reparaturenzym. Liposomenverkapselte Photolyase ist im Handel z. B. unter der Produktbezeichnung Photosomes™ (INCI-Bezeichnung: Aqua, Lecithin, Plankton Extract) liposomenverkapselte T4N5 z. B. unter der Bezeichnung Ultrasomes™ (INCI-Bezeichnung: Aqua, Lecithin, Micrococcus Lysate) von der Firma AGI Dermatics, USA, erhältlich. Da sich bei längerer Lagerung sowohl des Handelsprodukts als auch der erfindungsgemäßen Zusammensetzungen zwischen der verkapselten wässrigen, das Enzym enthaltenden Phase und der nicht-verkapselten, äußeren wässrigen Phase ein Gleichgewicht einstellt, liegt ein Teil der DNA-Reparaturenzyme unverkapselt vor.

In den erfindungsgemäßen Zusammensetzungen sind die Handelsprodukte Photosomes™ oder Ultrasomes™ bevorzugt in Mengen von 0,1 - 10 Gew.-%, besonders bevorzugt 0,5 - 5,0 Gew.-% und außerordentlich bevorzugt 1,0 -4,0 Gew.-%, bezogen auf das gesamte Mittel, enthalten. Bevorzugte erfindungsgemäße Zusammensetzungen sind **dadurch gekennzeichnet, dass** mindestens ein DNA-Reparaturenzym in Gesamtmengen von 0,00001 - 1 Gew.-%, bevorzugt 0,0001 - 0,1 Gew.-%, besonders bevorzugt 0,001 - 0,01 - 0,05 Gew.-% und außerordentlich bevorzugt 0,002 - 0,005 Gew.-%, enthalten ist.

Ganz besonders bevorzugte erfindungsgemäße Hautbehandlungsmittel sind dadurch gekennzeichnet, dass sie das/die DNA-Reparaturenzym(e) - bezogen auf das Gewicht des Hautbehandlungsmittels - in Mengen von 0,00001 - 1 Gew.-%, bevorzugt 0,0001 - 0,1 Gew.-%, besonders bevorzugt 0,001 -0,05 Gew.-% und außerordentlich bevorzugt 0,002 - 0,005 Gew.-%, enthalten. Die erfindungsgemäßen Zusammensetzungen können weiterhin die Pflanzenextrakte
- aus den Blüten von Lonicera japonica und/oder
- aus den Früchten von Xanthium sibiricum und/oder
- Extrakt aus den Wurzeln von Cyperus rotundus
enthalten. Ein Extrakt im Sinne der vorliegenden Anmeldung ist ein Stoff oder Stoffgemisch, welches durch Extraktion und teilweises oder völliges Eindampfen der Extraktionslösung gewonnen wurde. Man unterscheidet nach der Beschaffenheit *Trockenextrakte* d.h. bis zur Trockene eingedampfte Extrakte, *Fluidextrakte* d.h. mit Lösungsmitteln so hergestellte Extrakte, dass aus einem Teil Droge höchstens 2 Teile Fluid-Extrakt gewonnen werden, *zähflüssige Extrakte bzw. Dickextrakte,* d.h. Extrakte, bei denen ein Teil des Lösungsmittels verdampft wird.

Die erfindungsgemäß verwendeten Extrakte werden aus den jeweiligen Pflanzenteilen deren Gemischen durch Extraktion vorzugsweise mit organischen Lösemitteln, Wasser oder Gemischen daraus, gewonnen. Bevorzugt geeignete organische Lösemittel sind Ketone (z.B. Aceton), Ether, Ester, Alkohole oder halogenierte Kohlenwasserstoffe. Besonders bevorzugte Extraktionsmittel sind Wasser und/oder Alkohole. Unter den Alkoholen sind dabei (C₁ bis C₆)- Alkohole, wie Ethanol und Isopropanol und zwar sowohl als alleiniges Extraktionsmittel als auch in Mischung mit Wasser, bevorzugt. Besonders bevorzugte Extraktionsmittel sind Wasser, Ethanol, 2-Propanol, 1,2-Propylenglycol, 1,3-Butylenglycol, ganz besonders bevorzugt sind Wasser, Ethanol, 2-Propanol und 1,2-Propylenglycol sowie Mischungen hiervon, z. B. eine Mischung 1,2-Propylenglycol/Wasser im Verhältnis 4:1. Die Extraktion wird bevorzugt bei einer Temperatur von 25°C bis 90°C durchgeführt.

Besonders bevorzugte erfindungsgemäße kosmetische Mittel sind **dadurch gekennzeichnet, dass** der Extrakt mindestens ein polares Lösungsmittel, ausgewählt aus Ethanol, 1-Propanol, 2-Propanol, 1-Butanol, 2-Butanol, Ethylenglycol, 1,2-Propylenglycol, 1,3-Butylenglycol, Glycerin und Wasser, sowie Mischungen hiervon enthält.

Je nach Wahl der Extraktionsmittel kann es bevorzugt sein, den Extrakt durch Zugabe eines Lösungsvermittlers zu stabilisieren. Als Lösungsvermittler geeignet sind z. B. Ethoxylierungsprodukte von gegebenenfalls gehärteten pflanzlichen und tierischen Ölen. Bevorzugte Lösungsvermittler sind ethoxylierte Mono-, Di- und Triglyceride von C8-22-Fettsäuren mit 4 bis 50 Ethylenoxid-Einheiten, z. B. hydriertes ethoxyliertes Castoröl, Olivenölethoxylat, Mandelölethoxylat, Nerzölethoxylat, Polyoxyethylenglycolcapryl-/caprinsäureglyceride, Polyoxyethylenglycerinmonolaurat und Polyoxyethylenglycolkokosfettsäureglyceride. Besonders bevorzugt ist Olivenölethoxylat (INCI-Bezeichnung: PEG-10 Olive Glycerides).

Die Trockenmasse des Extrakts ist abhängig von der Molmasse und der Löslichkeit der dispergierten Inhaltsstoffe und beträgt in der Regel, jeweils bezogen auf das Gewicht des Extrakts, 1 - 80 Gew.-%. Bevorzugt beträgt die Trockenmasse 15 - 50 Gew.-% und besonders bevorzugt 20 - 35 Gew.-%. Bei einem Molekulargewicht der Inhaltsstoffe über 100.000 Dalton kann eine Trockenmasse von 1 - 20 Gew.-% bevorzugt und eine Trockenmasse von 1 - 10 Gew.-% besonders bevorzugt sein. Besonders bevorzugte kosmetische Mittel sind **dadurch gekennzeichnet, dass** die Trockenmasse des Extrakts 5 - 80 Gew.-%, bezogen auf das Gewicht des Extrakts, beträgt.

Die erfindungsgemäßen Zusammensetzungen enthalten vorzugsweise mindestens einen Extrakt aus den Blüten von Lonicera japonica ("Japanisches Geißblatt").Erfindungsgemäß bevorzugte Zusammensetzungen sind **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Blüten von Lonicera japonica enthalten.

Die erfindungsgemäßen Zusammensetzungen enthalten vorzugsweise mindestens einen Extrakt aus den Früchten von Xanthium sibiricum ("Spitzkletten").Erfindungsgemäß bevorzugte Zusammensetzungen sind **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Früchten von Xanthium sibiricum enthlten.

Die erfindungsgemäßen Zusammensetzungen enthalten vorzugsweise mindestens einen Extrakt aus den Wurzeln von Cyperus rotundus (Nagarmustaka). Erfindungsgemäß bevorzugte Zusammensetzungen sind **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Wurzeln von Cyperus rotundus enthalten.

Ganz besonders bevorzugte erfindungsgemäße Zusammensetzungen enthalten die drei Extrakte (mindestens einen Extrakt aus den Blüten von Lonicera japonica und mindestens einen Extrakt aus den Früchten von Xanthium sibiricum und mindestens einen Extrakt aus den Wurzeln von Cyperus rotundus) in einer Gesamtmenge von - bezogen auf das Gewicht der Zusammensetzung - 0,001 bis 15 Gew.-%, vorzugsweise 0,005 bis 12 Gew.-%, weiter bevorzugt 0,01 bis 10 Gew.-%, noch weiter bevorzugt 0,05 bis 7 Gew.-% und insbesondere 0,15 bis 6 Gew.-%. Anstelle der vorstehend genannten Verbindungen oder in Ergänzung zu ihnen können die erfindungsgemäßen Mittel auch Wirkstoffe enthalten, die die enzymatische Aktivität oder die Expression der L-Isoform der Lysyloxidase (LOXL) stimulieren.

Mit dem Begriff "LOXL" oder "hLOXL" ist die L-Isoform des humanen Proteins Lysyloxidase LOXL gemeint. Mit dem Begriff "LOX" oder "hLOX" ist die Anfangs-Isoform ("Pisoform initiale") des humanen Proteins Lysyloxidase LOX gemeint. Mit "Stimulieren der Expression der Isoform der Lysyloxidase LOXL" ist die Stimulierung der Expression des Gens, das für LOXL kodiert, oder von dessen Promotor gemeint, und insbesondere ist die Stimulierung der Synthese der messenger RNA, die für LOXL kodiert, und ebenfalls die Stimulierung der Synthese von LOXL, das aus dieser messenger RNA resultiert, gemeint. Mit "Stimulieren der Expression von Elastin der Isoform der Lysyloxidase LOXL" ist die Stimulierung der Expression des Gens, das für das Protein Elastin kodiert, oder von dessen Promotor gemeint, und insbesondere ist die Stimulierung der Synthese der messenger RNA, die für das Protein Elastin kodiert, und ebenfalls die Stimulierung der Synthese des Proteins Elastin oder seines Vorläufers, Tropoelastin, aus dieser messenger RNA resultiert, gemeint.

Wirkstoffe sind dann als wirksam anzusehen, wenn sie eine Aktivierung oder einen 1,5-fachen Anstieg der Expression und/oder der Aktivität von LOXL in einem Modell erzielen, welches mindestens einen Zelltyp umfasst, der eine Expression und/oder Aktivität von LOXL über den Kontakt mit diesen Wirkstoffen aufweist, in Bezug auf den Expressionslevel und/oder Aktivitätslevel von LOXL in einem Kontrollmodell (ohne Kontakt mit den Wirkstoffen). Vorteilhafterweise ist die Expression von LOXL entweder die Expression einer Nukleotidsequenz, die für LOXL kodiert, oder die Expression einer Aminosäuresequenz, die eine Teilsequenz des Proteins LOXL darstellt.

Vorteilhafterweise wird die Wirksubstanz ausgewählt aus der Gruppe
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten.

Anstelle der vorstehend genannten Verbindungen oder in Ergänzung zu ihnen können die erfindungsgemäßen Mittel auch Hyaluronsäure(Hyaluronan) enthalten. Hyaluronsäure ist ein Glycosaminoglycan, das im Bindegewebe, im Glaskörper, in der Nabelschnur und in der Synovialflüssigkeit der Gelenke vorkommt. Hyaluronsäure ist eine hochmolekulare Verbindung mit Molmassen zwischen 50000 und mehreren Millionen. Grundbaustein der Hyaluronsäure ist ein aus D-Glucuronsäure und N-Acetyl-D-glucosamin in β-(1, 3)-glycosidischer Bindung aufgebautes Aminodisaccharid, das mit der nächsten Einheit β-(1, 4)-glycosidisch verbunden ist.

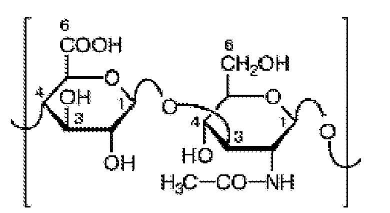

Die unverzweigte Kette der Hyaluronsäure besteht aus 2000-10000 solcher Einheiten. Hyaluronsäure-Ketten sind in Lösung helical angeordnet.

Anstelle der vorstehend genannten Verbindungen oder in Ergänzung zu ihnen können die erfindungsgemäßen Mittel auch Extrakte aus Apfelkernen enthalten.

Ein erfindungsgemäß besonders bevorzugter Apfelkernextrakt ist das Handelsprodukt Ederline des Herstellers Seporga. Ederline enthält Phytohormone, Isoflavonoide, Phytosterole, Triterpenoide, Tocopherole und natürliche Wachse. In vitro-Tests mit kultivierten Hautzellen zeigen laut Herstellerangaben Effekte auf die Synthese von Kollagen Typ I und Kollagen Typ III sowie auf Fibronectin. Desweiteren wurde in Probandenstudien eine positive Wirkung von Ederline auf das Hautrelief gemessen. Außerdem machen die Herstellerangaben antioxidative und antiinflammatorische Wirkungen geltend. Das Produkt Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich. Erfindungsgemäß geeignete Mengen an Ederline sind 0,1 - 10 Gew.-%, bevorzugt 1 - 8 Gew.-% und besonders bevorzugt 3 - 5 Gew.-%, jeweils bezogen auf die gesamte Zusammensetzung. Bezogen auf den Gehalt an aktiven Wirkstoffen, werden Apfelkernextrakte erfindungsgemäß in Mengen von 0,001 - 2 Gew.-%, bevorzugt 0,01 - 1,6 Gew.-% und besonders bevorzugt 0,03 - 1 Gew.-%, jeweils bezogen auf die gesamte Zusammensetzung, eingesetzt. Anstelle der vorstehend genannten Verbindungen oder in Ergänzung zu ihnen können die erfindungsgemäßen Mittel auch Phytohormone enthalten. Unter Phytohormonen (Pflanzenwuchsstoffen) werden im Rahmen der vorliegenden Erfindung Pflanzenwuchsstoffe aus den Gruppen der
- Derivate der 1*H*-Indol-3-ylessigsäure
- Gibberelline
- Cytokinine
- Abscisinsäure
verstanden.

In die erste Gruppe gehören z. B. die 2-Naphthyloxyessigsäure, Benzoesäure-Derivate, Phenoxycarbonsäure-Derivate usw.

Anstelle der vorstehend genannten Verbindungen oder in Ergänzung zu ihnen können die erfindungsgemäßen Mittel auch Isoflavonoide enthalten. Isoflavonoide sind zu den Flavonoiden zählende, gelegentlich auch als Isoflavone bezeichnete Gruppe von meist gelblich gefärbten Pflanzenfarbstoffen, die sich von Isoflavon ableiten. Der Grundkörper Isoflavon (3-Phenylchromon, 3-Phenyl-4H-1-benzopyran-4-on) kommt in Kleearten vor.

Einige bekanntere Isoflavone sind *Daidzein* (4',7-Dihydroxy-loflavon),*Genistein* (4',5,7-Trihydroxy-Isoflavion); *Prunetin* (4',5-Dihydroxy-7-methoxy-Isoflavon); *Biochanin A* (5,7-Dihydroxy-4'-methoxy-Isoflanon); *Orobol* (3',4',5,7-Tetrahydroxy-Isoflavon); *Santal* (3',4',5-Trihydroxy-7-methoxy-Isoflavon).

Anstelle der vorstehend genannten Verbindungen oder in Ergänzung zu ihnen können die erfindungsgemäßen Mittel auch Phytosterole enthalten. Phytosterole (Phytosterine) sind Sterole aus Pflanzen und Hefen (Mykosterole). Sie unterscheiden sich von Sterolen tierischen Ursprungs durch eine Doppelbindung an C-22 und C₁- od. C₂-Substituenten an C-24. Die häufigsten pflanzlichen Sterole sind Stigmasterol, β-Sitosterol und Campesterol.

Anstelle der vorstehend genannten Verbindungen oder in Ergänzung zu ihnen können die erfindungsgemäßen Mittel auch Triterpenoide enthalten. Triterpenoide (Triterpe) sind aus 6 Isopren-Einheiten aufgebaute Naturstoffe mit 30 Kohlenstoff-Atomen, die zur Gruppe der Terpene gehören. Zur Zeit sind rund 5000 Triterpene bekannt. Das einfachste Triterpen ist das acyclische Squalen, das auch die Muttersubstanz aller weiteren Triterpene ist. Monocyclische, bicyclische und tricyclische Triterpene sind sehr selten. Sie entstehen infolge einer gestörten Squalen-Cyclisierung, so z. B. das tricyclische Ambrein. Die Mehrzahl der Triterpene ist tetracyclisch bzw. pentacyclisch.

Anstelle der vorstehend genannten Verbindungen oder in Ergänzung zu ihnen können die erfindungsgemäßen Mittel auch Tocopherole enthalten. Tocopherole ist die Bezeichnung für in 2-Stellung mit einem 4,8,12-Trimethyltridecyl-Rest substituierte Chroman-6-ole (3,4-Dihydro-2H-1-benzopyran-6-ole).

Als eine weitere besonders geeignete Verbindung können die erfindungsgemäßen Mittel mindestens einen Stoff aus der Gruppe der Ellagsäure und/oder Ellagate enthalten. Ellagsäure (2,3,7,8-Tetrahydroxy[1]benzopyrano-[5,4,3-*cde*][1]benzopyran-5,10-dion 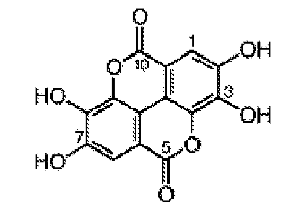 ist frei und in Form von Glycosiden und/oder Methylethern im Pflanzenreich weit verbreitet, besonders in Galläpfeln, Blattgallen, auch in den Blättern von *Eucalyptus*-Arten, in Eichen und Euphorbien.

Bevorzugte erfindungsgemäße Zusammensetzungen sind **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Ellagsäure (2,3,7,8-Tetrahydroxy[1]benzopyrano-[5,4,3-cde][1]benzopyran-5,10-dion) und/oder Ellagate enthalten.

Als eine weitere besonders geeignete Verbindung können die erfindungsgemäßen Mittel mindestens einen Stoff aus der Gruppe der Xanthophylle, insbesondere Astaxanthin, enthalten.

Xanthophylle ist der Gruppenname für Hydroxy-, Epoxy- und Oxo-Derivate von Carotinoiden (= Tetraterpenen mit einem C₄₀-Grundgerüst). Beispiele für erfindungsgemäß geeignete Verbindungen sind z. B. Canthaxanthin, Citranaxanthin, Flavoxanthin, Rhodoxanthin, Rubixanthin, Zeaxanthin, Lutein, Cryptoxanthin, Violaxanthin und insbesondere Astaxanthin. Die natürlich vorkommenden Xanthophylle sind meist *all-trans*-Verbindungen. Erfindungsgemäß besonders bevorzugt sind natürlich vorkommende Xanthophylle.

Weitere erfindungsgemäß bevorzugte Zusammensetzungen enthalten - bezogen auf ihr Gewicht - das mindestens eine Carotinoid in einer Gesamtmenge von 0,001 - 2 Gew.-%, bevorzugt 0,005 - 1 Gew.-%, besonders bevorzugt 0,01 - 0,5 Gew.-%, außerordentlich bevorzugt 0,05 - 0,2 Gew.-%. Erfindungsgemäß bevorzugte Zusammensetzungen enthalten - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-%, besonders bevorzugt 0,01 - 0,5 Gew.-%, außerordentlich bevorzugt 0,05 - 0,2 Gew.-% Xanthophylle, vorzugsweise Canthaxanthin, Citranaxanthin, Flavoxanthin, Rhodoxanthin, Rubixanthin, Zeaxanthin und ganz besonders bevorzugt Astaxanthin.

In einer weiteren bevorzugten Ausführungsform enthalten die erfindungsgemäßen Zusammensetzungen mindestens ein Vitamin, Provitamin oder eine als Vitaminvorstufe bezeichnete Verbindung aus den Vitamingruppen A, B, C und E und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*,10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-I) eingesetzt. 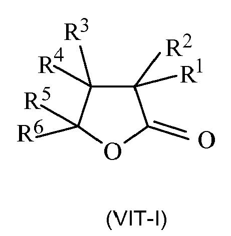 Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure. Erfindungsgemäß besonders bevorzugte Zusammensetzungen sind **dadurch gekennzeichnet, dass** sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind **dadurch gekennzeichnet, dass** sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Erfindungsgemäß besonders bevorzugte Zusammensetzungen sind **dadurch gekennzeichnet, dass** sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu).
   Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind **dadurch gekennzeichnet, dass** sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind **dadurch gekennzeichnet, dass** sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und aus Pantolacton. Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind **dadurch gekennzeichnet, dass** sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind **dadurch gekennzeichnet, dass** sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten. Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt. Vitamin A-palmitat (Retinylpalmitat), Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

Zusammenfassend sind erfindungsgemäße Zusammensetzungen bevorzugt, die zusätzlich - bezogen auf ihr Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C und E zugeordnet werden, wobei bevorzugte Mittel Panthenol ((±)-2,4-Dihydroxy-N-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.

Es sei an dieser Stelle darauf hingewiesen, dass sich die Angabe Gew.-%, sofern es nicht anders angegeben ist, jeweils auf das gesamte Mittel bezieht.

Der Gehalt an Wasser in bevorzugten erfindungsgemäßen Mitteln richtet sich u.a. danach, ob das Mittel in flüssiger oder fester Form vorliegt, beträgt daher vorzugsweise 0,5 bis 95 Gew.-%, wobei Werte von maximal 5 Gew.-% insbesondere bei festen oder nichtwässrigen flüssigen Mitteln besondere Bevorzugung finden.

Im Falle flüssiger Mittel enthält das erfindungsgemäße Mittel nach einer bevorzugten Ausführungsform Wasser in einer Menge von mehr als 20 Gew.-%, vorteilhafterweise mehr als 30 Gew.-%., in weiter vorteilhafter Weise mehr als 40 Gew.-%, noch vorteilhafter mehr als 50 Gew.-%, insbesondere 60 bis 99 Gew.-%, besonders bevorzugt 70 bis 98 Gew.-% und äußerst bevorzugt 80 bis 95 Gew.-%, bezogen auf das gesamte Mittel. Die Obergrenze an Wasser kann auch bei 80 Gew.-%, 70 Gew.-%, 60 Gew.-%, 50 Gew.-%, 40 Gew.-%, 30 Gew.-%, 20 Gew.-% oder 10 Gew.-% Gew.-% liegen, bezogen auf das gesamte Mittel.

Die Untergrenze an Wasser kann z.B. auch bei 80 Gew.-%, 70 Gew.-%, 60 Gew.-%, 50 Gew.-%, 40 Gew.-%, 30 Gew.-%, 20 Gew.-% oder 10 Gew.-% liegen, bezogen auf das gesamte Mittel. Die genannten Ober- und Untergrenzen können natürlich sinnvoll kombiniert werden, z.B. zu Wassergehalten von 60-80 Gew.-% oder 10-30 Gew.-% usw.

In einer weiteren bevorzugten Ausführungsform enthält das erfindungsgemäße Hautbehandlungsmittel zusätzlich mindestens eine UV-absorbierende Substanz. Bei einem Einsatz in geringen Mengen (bis etwa 1 Gew.-%) dient die UV-absorbierende Substanz dazu, vorteilhafterweise die Lichtbeständigkeit sonstiger Rezepturbestandteile zu verbessern (Produktschutz). Bei einem Einsatz in höheren Mengen (ab mehr als 1 Gew.-%) dient die UV-absorbierende Substanz dazu, die behandelte Haut vor den schädlichen Auswirkungen der UV-Strahlung zu schützen. Unter UV-absorbierende Substanz sind organische und anorganische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Verbindungen, die diese gewünschten Eigenschaften aufweisen, sind beispielsweise die durch strahlungslose Desaktivierung wirksamen Verbindungen und Derivate des Benzophenons mit Substituenten in 2- und/oder 4-Stellung. Weiterhin sind auch substituierte Benzotriazole, in 3-Stellung Phenylsubstituierte Acrylate (Zimtsäurederivate), gegebenenfalls mit Cyanogruppen in 2-Stellung, Salicylate sowie Naturstoffe wie Umbelliferon und die körpereigene Urocansäure geeignet. Besondere Bedeutung haben Biphenyl- und vor allem Stilbenderivate, kommerziell als Tinosorb^{®} FD oder Tinosorb^{®} FR ex Ciba erhältlich. Als UV-B-Absorber sind zu nennen 3-Benzylidencampher bzw. 3-Benzylidennorcampher und dessen Derivate, z.B. 3-(4-Methylbenzyliden)campher; 4-Aminobenzoesäurederivate, vorzugsweise 4-(Di-methylamino)benzoesäure-2-ethylhexylester, 4-(Dimethylamino)benzoesäure-2-octylester und 4-(Dimethylamino)benzoesäureamylester; Ester der Zimtsäure, vorzugsweise 4-Methoxyzimtsäure-2-ethylhexylester, 4-Methoxyzimtsäurepropylester, 4-Methoxyzimtsäureiso-amyl-ester, 2-Cyano-3,3-phenylzimtsäure-2-ethylhexylester (Octocrylene); Ester der Salicylsäure, vorzugsweise Salicylsäure-2-ethylhexylester, Salicylsäure-4-isopropylbenzyl-ester, Salicylsäurehomomenthylester; Derivate des Benzophenons, vorzugsweise 2-Hy-droxy-4-methoxybenzophenon, 2-Hydroxy-4-methoxy-4'-methylbenzophenon, 2,2'-Dihy-droxy-4-methoxybenzophenon; Ester der Benzalmalonsäure, vorzugsweise 4-Methoxy-benzmalonsäuredi-2-ethylhexylester; Triazinderivate, wie z.B. 2,4,6-Trianilino-(p-carbo-2'-ethyl-1'-hexyloxy)-1,3,5-triazin und Octyl Triazon, oder Dioctyl Butamido Triazone (Uvasorb® HEB); Propan-1,3-dione, wie z.B. 1-(4-tert.Butylphenyl)-3-(4'methoxyphenyl)propan-1,3-dion; Ketotricyclo(5.2.1.0)decan-Derivate. Weiterhin geeignet sind 2-Phenylbenzimidazol-5-sul-fonsäure und deren Alkali-, Erdalkali-, Ammonium-, Alkylammonium-, Alkanolammonium- und Glucammoniumsalze; Sulfonsäurederivate von Benzophenonen, vorzugsweise 2-Hy-droxy-4-methoxybenzophenon-5-sulfon-säure und ihre Salze; Sulfonsäurederivate des 3-Benzylidencamphers, wie z.B. 4-(2-Oxo-3-bor-nylidenmethyl)benzol-sulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), 1-Phenyl-3-(4'-isopropylphenyl)-propan-1,3-dion sowie Enaminverbindungen. Die UV-A und UV-B-Filter können selbstverständlich auch in Mischungen eingesetzt werden. Neben den genannten löslichen Stoffen kommen für diesen Zweck auch unlösliche Lichtschutzpigmente, nämlich feindisperse, vorzugsweise nanoisierte Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zink-stearat eingesetzt werden. Die Oxide und Salze werden in Form der Pigmente bereits für hautpflegende und hautschützende Emulsionen und dekorative Kosmetik verwendet. Die Partikel sollten dabei einen mittleren Durchmesser von weniger als 100 nm, vorzugsweise von 5 bis 50 nm und insbesondere von 15 bis 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000 (Merck). Als hydrophobe Coatingmittel kommen dabei vor allem Silicone und dabei speziell Trialkoxyoctylsilane oder Simethicone in Frage. Ebenfalls bevorzugt kann mikronisiertes Zinkoxid verwendet werden. Weitere geeignete UV-Lichtschutzfilter sind dem einschlägigen Stand der Technik zu entnehmen.

Die mindestens eine UV-absorbierende Substanz ist in bevorzugten erfindungsgemäßen Hautbehandlungsmitteln in einer Gesamtmenge von 0,01 Gew.-% bis 20 Gew.-%, besonders bevorzugt 1 - 10 Gew.-%, außerordentlich bevorzugt 2 Gew.-% bis 7 Gew.-%, enthalten.

Weitere bevorzugte erfindungsgemäße Zusammensetzungen sind **dadurch gekennzeichnet, dass** zur Verbesserung der Hautfeuchtigkeit unter Verzicht auf komedogene Substanzen mindestens ein alkyl- oder hydroxyalkylsubstituierten Harnstoff der allgemeinen Formel (UREA) enthalten ist, 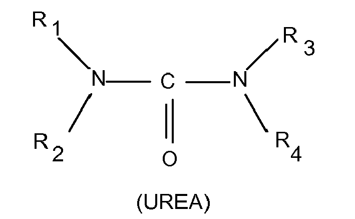 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hyd roxyalkyl-Gruppen substituiert ist, darstellt.

Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (UREA) ausgewählt ist aus Verbindungen, in denen R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-HydroxyalkylGruppen substituiert ist, stehen, mit der Maßgabe, dass mindestens einer der Reste R₁ und R₂ und gleichzeitig mindestens einer der Reste R₃ und R₄ eine C₂-C₆-Hydroxyalkyl-Gruppe, die mit 1 bis 5 Hydroxylgruppen oder C₁-C₄-Hydroxyalkyl-Gruppen substituiert ist, darstellt.

Weitere bevorzugte erfindungsgemäße Zusammensetzungen sind **dadurch gekennzeichnet, dass** der mindestens eine alkyl- oder hydroxyalkylsubstituierte Harnstoff der allgemeinen Formel (UREA) ausgewählt ist aus Bis-N,N'- (2-hydroxyethyl)harnstoff.

Erfindungsgemäß besonders bevorzugte Zusammensetzungen sind **dadurch gekennzeichnet, dass** sie mindestens einen alkyl- oder hydroxyalkylsubstituierten Harnstoff gemäß Formel (UREA), insbesondere N,N'-Bis-(2-hydroxyethyl)harnstoff, in einer Gesamtmenge von 0,01 - 50 Gew.-%, bevorzugt 0,1 - 20 Gew.-%, besonders bevorzugt 1 - 10 Gew.-% und außerordentlich bevorzugt 2 - 5 Gew.-%, bezogen auf die gesamte Zusammensetzung, enthalten. N,N'-Bis-(2-hydroxyethyl)harnstoff, bei Raumtemperatur ein kristalliner Feststoff, ist beispielsweise als Handelsprodukt Hydrovance von AkzoNobel als ca. 45 - 55 %ige wässrige Lösung mit einem Gehalt an Harnstoff und Ammoniumlactat erhältlich.

In einer weiteren bevorzugten Ausführungsform enthalten die erfindungsgemäßen Zusammensetzungen mindestens eine natürliche Betainverbindung. Erfindungsgemäße natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Erfindungsgemäß besonders bevorzugte Zusammensetzungen sind **dadurch gekennzeichnet, dass** sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.

In einer weiteren bevorzugten Ausführungsform enthalten die erfindungsgemäßen Zusammensetzungen neben der erfindungsgemäßen Wirkstoffkombination mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Zusammensetzungen sind **dadurch gekennzeichnet, dass** sie neben der erfindungsgemäßen Wirkstoffkombination mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt.

Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-a-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid). Erfindungsgemäß außerordentlich bevorzugte Flavonoide sind α-Glucosylrutin, Naringin und Apigenin-7-glucosid. Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon. Erfindungsgemäß besonders bevorzugte Zusammensetzungen sind **dadurch gekennzeichnet, dass** sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isoflavonoid-reichen Pflanzenextrakt. Zu den Isoflavonoiden werden an dieser Stelle die Isoflavone und die Isoflavon-Glycoside gezählt. Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin. In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.

Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter dem Namen Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse. Erfindungsgemäß besonders bevorzugte Zusammensetzungen sind **dadurch gekennzeichnet, dass** sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt.

Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein natürlich vorkommendes Xanthin-Derivat, ausgewählt aus Coffein, Theophyllin, Theobromin und Aminophyllin. Erfindungsgemäß bevorzugt sind die natürlich vorkommenden Xanthin-Derivate in Mengen von 0,0001 bis 1 Gew.-%, besonders bevorzugt 0,001 bis 0,5 Gew.-% und außerordentlich bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für N-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.

Erfindungsgemäß besonders bevorzugte kosmetische oder dermatologische Zusammensetzungen sind **dadurch gekennzeichnet, dass** sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Desoxyzucker oder ein mindestens einen Desoxyzucker-Baustein enthaltendes Polysaccharid.

Erfindungsgemäß bevorzugte Desoxyzucker sind ausgewählt aus Rhamnose und Fucose. Erfindungsgemäß besonders bevorzugte, Desoxyzucker-Bausteine enthaltende Polysaccharide sind die Polysaccharied mit der INCI-Bezeichnung Biosaccharide Gum-1, erhältlich beispielsweise als Handelsprodukt Fucogel^{®} von Solabia, Polysaccharide mit der INCI-Bezeichnung Biosaccharide Gum-2, erhältlich beispielsweise als Handelsprodukt Rhamnosoft^{®} von Solabia, Polysaccharide mit der INCI-Bezeichnung Biosaccharide Gum-3, erhältlich beispielsweise als Handelsprodukt Fucogenol^{®} von Solabia, und Polysaccharide mit der INCI-Bezeichnung Biosaccharide Gum-4, erhältlich beispielsweise als Handelsprodukt Glycofilm^{®} von Solabia, weiterhin Mischungen der vorgenannten, mindestens einen Desoxyzucker-Baustein enthaltenden Polysaccharide, beispielsweise die Mischung aus Biosaccharide Gum-2 und Biosaccharide Gum-3, erhältlich als Handelsprodukt Elastinol plus^{®} von Solabia.

Erfindungsgemäß besonders bevorzugte kosmetische oder dermatologische Zusammensetzungen sind **dadurch gekennzeichnet, dass** sie mindestens einen Desoxyzucker und/ oder mindestens ein Desoxyzucker-Bausteine enthaltendes Polysaccharid in Gesamtmengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Zusammensetzung, enthalten. Vorteilhafterweise liegen die erfindungsgemäßen Hautbehandlungsmittel in Form einer flüssigen, fließfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

In einer besonderen Ausführungsform der erfindungsgemäßen Mittel liegen die Mittel als Mikroemulsion vor. Unter Mikroemulsionen werden im Rahmen der Erfindung neben den thermodynamisch stabilen Mikroemulsionen auch die so genannten "PIT"-Emulsionen verstanden. Bei diesen Emulsionen handelt es sich um Systeme mit den 3 Komponenten Wasser, Öl und Emulgator, die bei Raumtemperatur als Öl-in-Wasser-Emulsion vorliegen. Beim Erwärmen dieser Systeme bilden sich in einem bestimmten Temperaturbereich (als Phaseninversiontemperatur oder "PIT" bezeichnet) Mikroemulsionen aus, die sich bei weiterer Erwärmung in Wasser-in-Öl-Emulsionen umwandeln. Bei anschließendem Abkühlen werden wieder O/W-Emulsionen gebildet, die aber auch bei Raumtemperatur als Mikroemulsionen oder als sehr feinteilige Emulsionen mit einem mittleren Teilchendurchmesser unter 400 nm und insbesondere von etwa 100-300 nm, vorliegen. Erfindungsgemäß können solche Mikro- oder "PIT"-Emulsionen bevorzugt sein, die einen mittleren Teilchendurchmesser von etwa 200 nm aufweisen.

Ein weiterer Gegenstand der vorliegenden Erfindung ist die Verwendung von erfindungsgemäßen Zusammensetzungen zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut.

Bezüglich weiterer bevorzugter Ausführungsformen der erfindungsgemäßen Verwendungen gilt mutatis mutandis das zu den erfindungsgemäßen Mitteln Gesagte.

Ein weiterer Gegenstand der vorliegenden Erfindung ist ein nicht-therapeutisches Verfahren zur nicht-therapeutischen, kosmetischen Behandlung und/oder Minimierung von Hautfalten und -fältchen, den Anzeichen der intrinsischen und extrinsischen Hautalterung, müder und/oder schlaffer Haut, UV-geschädigter Haut und/oder gereizter Haut, **dadurch gekennzeichnet, dass** ein erfindungsgemäßes Hautbehandlungsmittel auf die Haut aufgetragen wird.

Auch bezüglich weiterer bevorzugter Ausführungsformen der erfindungsgemäßen Verfahren gilt mutatis mutandis das zu den erfindungsgemäßen Mitteln Gesagte.

### Beispiele:

### Beispiel 1: Anreicherung epidermaler Stammzellen

Epidermale Stammzellen wurden basierend auf dem von Jones & Watt beschriebenen Verfahren isoliert (Jones P.H., Watt F.M., Seperation of Human Epidermal Stem Cells from Transit Amplifying Cells on the Basis of Difference in Integrin Function and Expression (1993) Cell 73, 713-724). Bei diesem Verfahren werden Keratinozyten zunächst aus der Haut isoliert und anschließend auf mit Kollagen Typ IV beschichtete Zellkulturplatten ausgesät. Zellen, die innerhalb von 20 Minuten auf diesen Platten adhärieren, sind angereichert mit epidermalen Stammzellen. Die noch im Überstand befindlichen Zellen bestehen zum größten Teil aus den so genannten "Transit Amplifying Cells" (TA) und "Post-mitotic Cells"(PM) und können durch Abnahme der Überstandes von der Stammzell-angereicherten Kultur abgetrennt werden. Zum Nachweis der epidermalen Stammzellen nach dem Trennungsprozess können verschiedene Marker herangezogen werden. So wird das Protein CD71 auf epidermalen Stammzellen in einem geringeren Maße exprimiert als auf TA-Zellen. Das β1-Integrin ist auf epidermalen Stammzellen wiederum stärker exprimiert als auf TA- und PM-Zellen. Die Marker wurden zur Charakterisierung der Stammzellpräparationen vor der Durchführung der im Folgenden beschriebenen Experimente herangezogen. Für die Wirkstoffbehandlung und Bestrahlung wurden die angereicherten epidermalen Stammzellen ohne weitere Passage in einer Dichte von etwa 80.000 Zellen pro Vertiefung in einer mit Kollagen Typ IV beschichteten 96 *wellplate* ausgesät.

### Beispiel 2: Zellvitalität nach Bestrahlung

Zur Untersuchung der zellschützenden Wirkung verschiedener Substanzen wurden Präparationen epidermaler Stammzellen mit den jeweiligen Substanzen für 4 Stunden vorinkubiert und anschließend, bei weitergeführter Wirkstoffinkubation, mit 10 J/cm² UVA bestrahlt (Hönle, SOL500, Filter H2). Nach einer Erholungsphase von weiteren 18 h unter Wirkstoffeinwirkung wurde mit Hilfe eines MTT-Assays die Vitalität der Zellen bestimmt. Der applizierte UV-Stress führte zu einer Abnahme der Vitalität der epidermalen Stammzellen. Dieser Effekt konnte allerdings durch die Inkubation mit einer Kombination aus Lipochroman-6 und einem Malus Domestica-Extrakt (PhytoCellTec) verhindert werden.

| **Behandlung** | **Relative Vitalität** | **Stdabw.** |
|---|---|---|
| Kontrolle unbestrahlt | 1,24 | 0,16 |
| Kontrolle UVA-bestrahlt | 1,00 | 0,00 |
| Lipochroman-6 [30µM] + Malus Domestica [0,01%] | 1,25 | 0,11 |
| Lipochroman-6 [30µM] + Malus Domestica [0,05%] | 1,31 | 0,15 |
| Lipochroman-6 [30µM] + Malus Domestica [0,2%] | 1,31 | 0,15 |

Der beobachtete Effekt kann nicht allein auf die Wirkung des Malus Domestica-Extraktes (PhytoCellTec) zurückgeführt werden:

| **Behandlung** | **Relative Vitalität** | **Stdabw.** |
|---|---|---|
| Kontrolle unbestrahlt | 1,56 | 0,40 |
| Kontrolle UVA-bestrahlt | 1,00 | 0,00 |
| Malus Domestica [0,01%] | 0,98 | 0,11 |
| Malus Domestica [0,05%] | 1,11 | 0,21 |
| Malus Domestica [0,2%] | 1,05 | 0,13 |
| Malus Domestica [1,0%] | 1,01 | 0,10 |
| Malus Domestica [2,0%] | 0,99 | 0,12 |

### Beispiel 3: erfindungsgemäße Zusammensetzungen

### 1. Beispielserie:

| | **1** | **2** | **3** | **4** | **5** |
|---|---|---|---|---|---|
| Distelöl | 3,00 | 3,00 | 3,00 | 3,00 | 3,00 |
| Myritol 318 | 5,00 | 5,00 | 5,00 | 5,00 | 5,00 |
| Novata AB | 2,00 | 2,00 | 2,00 | 2,00 | 2,00 |
| Lanette 22 | 1,00 | 1,00 | 1,00 | 1,00 | 1,00 |
| Cutina MD | 2,00 | 2,00 | 2,00 | 2,00 | 2,00 |
| Stenol 1618 | 1,00 | 1,00 | 1,00 | 1,00 | 1,00 |
| Baysilon M350 | 1,00 | 1,00 | 1,00 | 1,00 | 1,00 |
| Controx KS | 0,05 | 0,05 | 0,05 | 0,05 | 0,05 |
| Propylparaben | 0,20 | 0,20 | 0,20 | 0,20 | 0,20 |
| Dry Flo Plus | 1,00 | 2,00 | 3,00 | 2,00 | 3,00 |
| Propylenglycol | 5,00 | 5,00 | 5,00 | 5,00 | 5,00 |
| Glycerine | 5,00 | 3,00 | 3,00 | 3,00 | 3,00 |
| Methylparaben | 0,20 | 0,20 | 0,20 | 0,20 | 0,20 |
| Phenoxyethanol | 0,9 | 0,5 | 0,9 | 0,5 | 0,9 |
| Parfum | 0,10 | 0,10 | 0,10 | 0,10 | 0,10 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Aqua | ad 100 | ad 100 | ad 100 | ad 100 | ad 100 |

### 2. Beispielserie:

| | **1** | **2** | **3** | **4** | **5** |
|---|---|---|---|---|---|
| Lipoid S75-3 | 0,50 | 0,50 | 0,50 | 0,50 | 0,50 |
| Tocopherylacetat | 0,50 | 0,50 | 0,50 | 0,50 | 0,50 |
| Cutina MD | 1,00 | 1,00 | 1,00 | 1,00 | 1,00 |
| Lanette 22 | 2,00 | 2,00 | 2,00 | 2,00 | 2,00 |
| Baysilone M 350 | 0,50 | 0,50 | 0,50 | 0,50 | 0,50 |
| Propylparaben | 0,20 | 0,20 | 0,20 | 0,20 | 0,20 |
| Dow Corning 9040 | 1,00 | 1,00 | 1,00 | 1,00 | 1,00 |
| Glycerin | 4,50 | 3,00 | 3,00 | 3,00 | 4,50 |
| Hexandiol | 6,00 | 3,00 | | 3,00 | 6,00 |
| Methylparaben | 0,20 | 0,20 | 0,20 | 0,20 | 0,20 |
| Tego Carbomer 140 | 0,30 | 0,30 | 0,30 | 0,30 | 0,30 |
| DS-HCN | 5,00 | 5,00 | 5,00 | 5,00 | 5,00 |
| Propylenglycol | 5,00 | 5,00 | 5,00 | 5,00 | 5,00 |
| Simugel NS | 1,50 | 1,50 | 1,50 | 1,50 | 1,50 |
| TiO2 | 0,50 | 0,50 | 0,50 | 0,50 | 0,50 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Wasser | ad 100 | ad 100 | ad 100 | ad 100 | ad 100 |

### 3 Beispielserie:

| | **1** | **2** | **3** | **4** | **5** |
|---|---|---|---|---|---|
| Sisterna SP30-C | 2,0 | 2,0 | 2,0 | 2,0 | 2,0 |
| Sisterna SP70-C | 0,8 | 0,8 | 0,8 | 0,8 | 0,8 |
| Propylparaben | 0,2 | 0,2 | 0,2 | 0,2 | 0,2 |
| Methylparaben | 0,2 | 0,2 | 0,2 | 0,2 | 0,2 |
| Ethanol | 5,0 | 3,0 | | 5,0 | 3,0 |
| Dry Flo Plus | 1,0 | 1,0 | 1,0 | 1,0 | 1,0 |
| Parfum | 0,3 | 0,3 | 0,3 | 0,3 | 0,3 |
| Cetearyl Alcohol | 1,0 | 1,0 | 1,0 | 1,0 | 1,0 |
| Fucogel 1000 | 2,0 | 1,0 | 0,5 | 2,0 | 1,0 |
| Keltrol SF | 0,2 | | 0,2 | 0,2 | |
| Aristoflex AVC | | 0,5 | | | 0,5 |
| Hexandiol-1,6 | 5,0 | | | 5,0 | |
| Glycerin | 10,0 | 5,0 | 10,0 | 10,0 | 5,0 |
| Dow Corning 200 Fluid | 7,0 | | | 7,0 | |
| Dow Corning 245 | | 5,0 | 5,0 | | 5,0 |
| Cetiol 868 | 2,0 | | 2,0 | 2,0 | |
| Parfum | 0,3 | 0,3 | 0,3 | 0,3 | 0,3 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Wasser | ad 100 | ad 100 | ad 100 | ad 100 | ad 100 |

### 4 Beispielserie:

| | **1** | **2** | **3** | **4** | **5** |
|---|---|---|---|---|---|
| Dow Corning 245 Fluid | 25 | 25 | 25 | 25 | 25 |
| Abil EM 90 | 2 | 2 | 2 | 2 | 2 |
| Belsil DM 100 | 2,5 | 2,5 | 2,5 | 2,5 | 2,5 |
| Hydrogenated Castor Oil | 2 | 2,5 | 2 | 2 | 2,5 |
| Glycerin | 5 | 5 | 5 | 5 | 5 |
| NaCl | 2 | 2 | 2 | 2 | 2 |
| Symdiol 68 | 0,5 | 0,5 | 0,5 | 0,5 | 0,5 |
| Phenoxyethanol | 0,4 | 0,4 | 0,4 | 0,4 | 0,4 |
| Parfum | 0,3 | 0,3 | 0,3 | 0,3 | 0,3 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Wasser | ad 100 | ad 100 | ad 100 | ad 100 | ad 100 |

### 5. Beispielserie:

| | **1** | **2** | **3** | **4** | **5** |
|---|---|---|---|---|---|
| Montanov 202 | 5,0 | 5,0 | 5,0 | 5,0 | 5,0 |
| Cutina MD | 2,0 | 2,0 | 2,0 | 2,0 | 2,0 |
| Cetearyl Alcohol | 1,0 | 1,0 | 1,0 | 1,0 | 1,0 |
| Cetiol B | 9,0 | 9,0 | 9,0 | 9,0 | 9,0 |
| Controx KS | 0,05 | 0,05 | 0,05 | 0,05 | 0,05 |
| Dow Corning 245 | | 7,0 | 3,0 | | 7,0 |
| Dow Corning 9040 | 1,0 | 1,0 | | 1,0 | 1,0 |
| Parfum | 0,3 | 0,3 | 0,3 | 0,3 | 0,3 |
| Fucogel 1000 | | 2,0 | | | 2,0 |
| Keltrol SF | | 0,2 | 0,2 | | 0,2 |
| Aristoflex AVC | | | 0,5 | | |
| Hexandiol-1,6 | 6,0 | 6,0 | | 6,0 | 6,0 |
| Glycerin | 3,0 | 5,0 | 10,0 | 3,0 | 5,0 |
| Dow Corning 200 Fluid | 7,0 | 7,0 | | 7,0 | 7,0 |
| Dow Corning 245 | | 5,0 | 3,0 | | 5,0 |
| Dow Corning 9040 | 1,0 | | 1,0 | 1,0 | |
| Propylenglycol | 2,0 | 2,0 | | 2,0 | 2,0 |
| Tego Carbomer | 0,3 | 0,3 | 0,3 | 0,3 | 0,3 |
| Dry Flo Plus | 1,0 | 1,0 | 1,0 | 1,0 | 1,0 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Wasser | ad 100 | ad 100 | ad 100 | ad 100 | ad 100 |

### 6. Beispielserie:

| | **1** | **2** | **3** | **4** | **5** |
|---|---|---|---|---|---|
| Glucamate SSE-20 | 2,0 | 2,0 | 2,0 | 2,0 | 2,0 |
| Sympatens-BS/080 | 2,0 | 2,0 | 2,0 | 2,0 | 2,0 |
| Dry Flo Plus | | 1,0 | 1,0 | | 1,0 |
| Parfum | 0,3 | 0,3 | 0,3 | 0,3 | 0,3 |
| Silikonöl 350 | 0,3 | 0,3 | | 0,3 | |
| Cutina MD | 2,0 | 2,0 | 2,0 | 2,0 | 2,0 |
| Paraffinöl | 2,0 | 1,0 | | 2,0 | |
| Cetearyl Alcohol | 1,0 | 1,5 | 1,25 | 1,0 | 1,25 |
| Capryl-/Caprinsäure-Triglycerid | | 3,5 | 3,0 | | 3,0 |
| Phenoxyethanol | 0,98 | 0,5 | 0,9 | 0,98 | 0,9 |
| Keltrol SF | | | 0,2 | | 0,2 |
| Aristoflex AVC | | 0,5 | 0,5 | | 0,5 |
| Hexandiol-1,6 | | 5,0 | 5,0 | | 5,0 |
| Glycerin | 2,0 | 5,0 | 10,0 | 2,0 | 10,0 |
| Culminal MHPC 100 | 0,1 | 0,1 | 0,1 | 0,1 | 0,1 |
| Propylenglycol | 2,0 | 5,0 | 3,0 | 2,0 | 3,0 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Wasser | ad 100 | ad 100 | ad 100 | ad 100 | ad 100 |

### 7. Beispielserie:

| | **1** | **2** | **3** | **4** | **5** |
|---|---|---|---|---|---|
| Emulsiphos 677660 | 3 | 3 | 3 | 3 | 3 |
| Cetiol B | 5 | 5 | 5 | 5 | 5 |
| Estasan GT8-60 3575 MCT Oil | 3,5 | 3,5 | 3,5 | 3,5 | 3,5 |
| Safloröl raffiniert | 2 | 2 | 2 | 2 | 2 |
| Behenylalkohol | 3 | 3 | 3 | 3 | 3 |
| Cetiol Sensoft | 1 | 1 | 1 | 1 | 1 |
| Vitamin E Acetat | 0,5 | 0,5 | 0,5 | 0,5 | 0,5 |
| Controx KS | 0,05 | 0,05 | 0,05 | 0,05 | 0,05 |
| Bienenwachs | 0,2 | 0,2 | 0,2 | 0,2 | 0,2 |
| COSMEDIA SP | 0,5 | 0,5 | 0,5 | 0,5 | 0,5 |
| Butylmethoxydibenzoylmethan | 3 | 3 | 3 | 3 | 3 |
| Parsol SLX | 3 | 3 | 3 | 3 | 3 |
| Propylparaben | 0,2 | 0,2 | 0,2 | 0,2 | 0,2 |
| Hexandiol-1,6 | 5 | 5 | 5 | 5 | 5 |
| Glycerin 86% pflanzlich | 4,5 | 4,5 | 4,5 | 4,5 | 4,5 |
| Sorbit 70% LS DAB | 2 | 2 | 2 | 2 | 2 |
| Methylparaben | 0,2 | 0,2 | 0,2 | 0,2 | 0,2 |
| Tego Carbomer 140 | 0,5 | 0,5 | 0,5 | 0,5 | 0,5 |
| NTA-Na3 flüssig 40% | 0,1 | 0,1 | 0,1 | 0,1 | 0,1 |
| Cosmedia Silc | 2 | 2 | 2 | 2 | 2 |
| AMP Ultra PC 1000 | 0,99 | 0,99 | 0,99 | 0,99 | 0,99 |
| Neo Heliopan Hydro | 3 | 3 | 3 | 3 | 3 |
| Natriumhydroxid Perlen | 0,365 | 0,365 | 0,365 | 0,365 | 0,365 |
| Vilastene | 4 | 2 | 3 | 2 | 3 |
| Superoxiddismutase | 0,1 | | | | |
| Lipochroman | | 0,01 | | 0,01 | 0,02 |
| Phytocelltec Malus Domestica | | | 3 | 2 | |
| Wasser, vollentsalzt | ad 100 | ad 100 | ad 100 | ad 100 | ad 100 |

### Verzeichnis der eingesetzten Rohstoffe:

| | |
|---|---|
| Myritol 318 | INCI-Bezeichnung: Caprylic/Capric Triglyceride (Cognis) |
| Abil^{®} EM 90 | modifiziertes Polyether-Polysiloxan (INCI-Bezeichnung: Cetyl PEG/PPG-10/1 Dimethicone) (Evonik Degussa) |
| AMP Ultra PC 2000 | 2-Amino-2-methyl-propanol (ca. 94,5-95,4% Aktivsubstanz in Wasser; INCI-Bezeichnung: Aminomethyl Propanol) (Dow Chemical) |
| Aristoflex^{®} AVC | Vinylpyrrolidon/Acryloyldimethyltaurat-Copolymer (ca. 92% Festkörper) INCI-Bezeichnung : Ammonium Acryloyldimethyltaurate/VP Copolymer, t-Butyl Alcohol (Clariant) |
| Belsil DM 100 | Polydimethylsiloxan, INCI-Bezeichnung Dimethicone (Wacker) |
| Cetiol^{®} 868 | Ethylhexylstearat (INCI-Bezeichnung: Ethyl Hexyl Stearate) (Cognis) |
| Cetiol^{®} B | Dibutyladipat (Cognis) |
| Cetiol Sensoft | Propylheptyl Caprylate (Cognis) |
| Controx KS | Tocopherol, Hydrogenated Palm Glycerides Citrate (Cognis) |
| Cosmedia SP | Natriumpolyacrylat (Cognis) |
| Cosmedia Silc | Silica (Cognis) |
| Culminal MHPC 100 | INCI-Bezeichnung HYDROXYPROPYL METHYLCELLULOSE (Hercules) |
| Cutina^{®} MD | Glycerinmono/distearat (INCI-Bezeichnung: Glyceryl Stearate) (Cognis) |
| Dow Corning^{®} 200 Fluid | Polydimethylsiloxan (INCI-Bezeichnung: Dimethicone) (Dow Corning) |
| Dow Corning^{®} 245 Fluid | Dimethylsiloxanpentamer (95% Reinheit; INCI-Bezeichnung: Cyclomethicone) (Dow Corning) |
| Dow Corning 9040 | Cyclomethicone/ Dimethicone Crosspolymer (Dow Corning) |
| DSH-C N | Dimethylsilanolhyaluronat; wässrige Lösung, konserviert (1kg DSH-C enthält 3.0g Dimethylsilandiol, 15g Mucopolysaccharide, 1.5g Natriumhyaluronat, Konservierungsmittel: 0.128% Methylparaben-Natrium-Salz, 0.032% Propylparaben, 0.200% Imidazolidinyl-harnstoff; INCI-Bezeichnung: Dimethylsilanol Hyaluronate) (Exsymol) |
| Emulsiphos 677660 | Potassium Cetyl Phosphate, Hydrogenated Palm Glycerides (Symrise) |
| Estasan GT8-60 3575 | Caprylic acid, capric acid triglyceride (Uniqema) |
| Fucogel 1000 | INCI-Bezeichnung: Aqua, Biosaccharide Gum-1 (Solabia) |
| Glucamate SSE-20 | Poly(oxy-1,2-ethanediyl), alpha-hydro-omega-hydroxyether mit Methyl D-glucopyranosid (4:1), octadecanoate (2:3), INCI: PEG-20 METHYL GLUCOSE SESQUISTEARATE (Noveon) |
| Keltrol SF | Xanthan Gum (Kelco) |
| Lipoid S 75-3 | Aqua, Lecithine (Lipoid GmbH) |
| Montanov^{®} 202 | INCI-Bezeichnung: Arachidyl Alcohol, Behenyl Alcohol, Arachidyl Glucoside (Seppic) |
| Neo Heliopan^{®} Hydro | 2-Phenylbenzimidazol-2-sulfonsäure (INCI: Phenylbenzimidazole Sulfonic Acid) (Symrise) |
| Parsol^{®} SLX | Dimethicodiethylbenzal malonat (INCI-Bezeichnung: Polysilicone-15, CAS-Nr.: 207574-74-1) (DSM Nutritional Products) |
| Silikonöl 350 | Dimethicone |
| Simulgel NS | Hydroxyethyl Acrylate / Sodium Acryloyldimethyl Taurate Copolymer / Squalane / Polysorbate 60 (Seppic) |
| Sisterna SP 30 C | INCI-Bezeichnung: Sucrose Distearate (Sisterna B.V.) |
| Sisterna SP 70 C | INCI-Bezeichnung: Sucrose Stearate (Sisterna B.V.) |
| Symdiol 68 | 1,2-Octandiol, 1,2-Hexandiol (Symrise) |
| Sympatens-BS/080 | INCI-Bezeichnung PEG-8 STEARATE (Dr. Kolb) |
| Tego Carbomer 140 | Carboxyvinylpolymer (INCI-Bezeichnung: Carbomer) (Goldschmidt) |
| Vilastene^{®} | INCI-Bezeichnung: Water (Aqua), Lysine HCL, Lecithin, Caprylyl Glycol, Tripeptide-10 Citrulline, Carbomer, Sodium Hydroxide (Centerchem) |
| Baysilon M350 | Dimethicone |
| Lanette 22 | Behenylalkohol (Cognis) |
| Stenol^{®} 1618 | C₁₆₋₁₈-Fettalkohol (INCI-Bezeichnung: Cetearyl Alcohol) (Cognis) |
| Dry Flo Plus | Aluminium Starch Octenylsuccinate (National Starch) |
| Novata AB | Cocoglycerides (Cognis) |

## Patentansprüche

1. Kosmetische oder dermatologische topische Zusammensetzungen, enthaltend in einem geeigneten kosmetischen oder dermatologischen Träger
a) mindestens eine Substanz, ausgewählt aus 6,7-disubstituierten 2,2-Dialkylchromanen oder -chromenen der allgemeinen Formeln (I) oder (II), 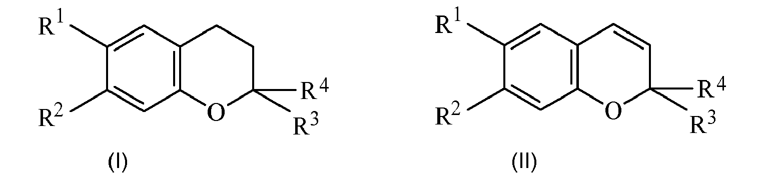 wobei R¹ und R² unabhängig voneinander eine OH-Gruppe, eine Methoxy-Gruppe oder eine CF₃CH₂O-Gruppe und R³ und R⁴ unabhängig voneinander eine C₁-C₄-Alkylgruppe darstellen, insbesondere Lipochroman-6
b) mindestens eine dedifferenzierte Pflanzenzellsuspension.

2. Zusammensetzung gemäß Anspruch 1, **dadurch gekennzeichnet, dass** sie - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% 6,7-disubstituierte 2,2-Dialkylchromane oder -chromene der allgemeinen Formeln (II) oder (III) wie vorstehend definiert, enthält, wobei bevorzugte Zusammensetzungen - bezogen auf ihr Gewicht - 0,0001 bis 10 Gew.-%, vorzugsweise 0,0005 bis 7,5 Gew.-%, weiter bevorzugt 0,001 bis 5 Gew.-%, noch weiter bevorzugt 0,002 bis 2,5 Gew.-% und insbesondere 0,005 bis 2 Gew.-% Lipochroman-6 enthalten.

3. Zusammensetzung nach einem der Ansprüche 1 oder 2, **dadurch gekennzeichnet, dass** sie mindestens eine dedifferenzierte Pflanzenzellsuspension von Pflanzen der Unterfamilie Maloideae (Kernobstgewächse) enthält.

4. Zusammensetzung nach einem der Ansprüche 1 bis 3, **dadurch gekennzeichnet, dass** sie mindestens eine dedifferenzierte Pflanzenzellsuspension von Pflanzen der Gattung Malus (Äpfel) enthält, wobei bevorzugte Pflanzenzellen ausgewählt sind aus Zellen von
■ *alus aldenhamensis*
■ *Malus angustifolia*
■ *Malus* × *arnoldiana* (*M. baccata* × *M. floribunda*)
■ *Malus asiatica*
■ *Malus* × *atrosanguineum* (*M. halliana* × M*. sieboldii*)
■ *Malus baccata*
■ *Malus bracteata*
■ *Malus brevipes*
■ *Malus coronaria*
■ *Malus daochengensis*
■ *Malus dasyphylia*
■ *Malus* × *domestica*
■ *Malus doumeri* (Bois) A.Chevalier
■ *Malus florentina*
■ *Malus floribunda*
■ *Malus formosana*
■ *Malus fusca*
■ *Malus glabrata*
■ *Malus glaucescens*
■ *Malus halliana*
■ *Malus hilleri*
■ *Malus honanensis*
■ *Malus hupehensis*
■ *Malus ioensis*
■ *Malus kansuensis*
■ *Malus komarovii*
■ *Malus lancifolia*
■ *Malus leiocalyca*
■ *Malus* × *magdeburgensis*
■ *Malus mandschurica*
■ *Malus melliana*
■ *Malus* × *micromalus* Makino
■ *Malus niedzwetskyana*
■ *Malus ombrophila*
■ *Malus platycarpa*
■ *Malus praecox*
■ *Malus prattii*
■ *Malus prunifolia*
■ *Malus pumila* Mill.
■ *Malus* × *purpurea*), (*M. atrosanguinea* × *M. niedzwetskyana*)
■ *Malus rockii*
■ *Malus sargentii* (Syn.: *M. toringo* ssp. *sargentii*)
■ *Malus sieboldii* (Syn.: *M. toringo*)
■ *Malus* × *cheideckeri*
■ *Malus sikkimensis*
■ *Malus sieversii*
■ *Malus* × *soulardi*
■ *Malus spectabilis*
■ *Malus sublobata*
■ *Malus sylvestris*
■ *Malus toringo*
■ *Malus toringoides*
■ *Malus transitoria*
■ *Malus trilobata*
■ *Malus tschonoskii*
■ *Malus yunnanensis*

5. Zusammensetzung nach einem der Ansprüche 1 bis 4, **dadurch gekennzeichnet, dass** sie mindestens eine dedifferenzierte Pflanzenzellsuspension von Pflanzen von Malus domestica Cultivar Uttwiler Spätlauber (Apfel Sorte Uttwiler Spätlauber) enthalten.

6. Zusammensetzung nach einem der Ansprüche 1 bis 5, **dadurch gekennzeichnet, dass** sie - bezogen auf ihr Gewicht - 0,001 bis 20 Gew.-%, vorzugsweise 0,002 bis 17,5 Gew.-%, weiter bevorzugt 0,005 bis 15 Gew.-%, noch weiter bevorzugt 0,01 bis 10 Gew.-% und insbesondere 0,05 bis 7 Gew.-% Pflanzenzellsuspension(en) enthält.

7. Zusammensetzung nach einem der Ansprüche 1 bis 6, **dadurch gekennzeichnet, dass** sie - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 2 Gew.-% Superoxiddismutase(n) enthält.

8. Zusammensetzung nach einem der Ansprüche 1 bis 7, **dadurch gekennzeichnet, dass** sie - bezogen auf ihr Gewicht - 0,001 bis 10 Gew.-% mindestens eines Wirkstoffes aus der Gruppe
- der Monomeren, Oligomeren und Polymeren von Aminosäuren, N-C₂-C₂₄-Acylaminosäuren, den Estern und/oder den physiologisch verträglichen Metallsalzen dieser Substanzen und/oder
- der Extrakte aus der Gruppe, bestehend aus Dill (Peucedanum Graveolens), Korinthe (Johannisbeere), Kardamom (Elettaria cardamomum), schwarzem Rettich, kleiner Stechpalme, Zimt, Hafer (Avena sativa), Kartoffel, Seide, und Asea foetida gum und/oder
- der Extrakte aus den Blüten von Lonicera japonica und/oder
- der Extrakte aus den Früchten von Xanthium sibiricum und/oder
- der Extrakte aus den Wurzeln von Cyperus rotundus und/oder
- Apfelkernextrakt und/oder
- Hyaluronsäure und/oder
- der Phytohormone und/oder
- der Isoflavonoide und/oder
- der Phytosterole und/oder
- der Triterpenoide und/oder
- der Tocopherole
- Ellagsäure und/oder Ellagate und/oder
- der Xanthophylle, insbesondere Astaxanthin, und/oder
- Milchsäurebakterien-basierten Fermentationen und/oder
- Ethylhexenoat und seinen Derivaten und/oder
- Methylbutyrat und seinen Derivaten und/oder
- Ethyldecadienoat und seinen Derivaten
enthält.

9. Zusammensetzung nach einem der Ansprüche 1 bis 8, **dadurch gekennzeichnet, dass** die Oligomeren von Aminosäuren, N-C₂-C₂₄-Acylaminosäuren und/oder den physiologisch verträglichen Metallsalzen dieser Substanzen ausgewählt sind aus Di-, Tri-, Tetra-, Penta-, Hexa- oder Pentadecapeptiden, die N-acyliert und/oder verestert sein können.

10. Zusammensetzung nach Anspruch 9, **dadurch gekennzeichnet, dass** das Oligopeptid ausgewählt ist aus Tyr-Arg und seinen N-acylierten Derivaten, insbesondere N-Acetyl-Tyr-Arg-hexyldecylester, Gly-His-Lys und seinen N-acylierten Derivaten, insbesondere N-Palmitoyl-Gly-His-Lys, Gly-His-Arg, und seinen N-acylierten Derivaten, insbesondere N-Myristoyl-Gly-His-Arg, Lys-Val-Lys und seinen N-acylierten Derivaten, insbesondere Palmitoyl-Lys-Val-Lys, Val-Tyr-Val, Gly-Gln-Pro-Arg (Rigin), Rigin-Analoga und Rigin-Derivaten, insbesondere N-Palmitoyl-Gly-Gln-Pro-Arg, Lys-Thr-Thr-Lys-Ser und seinen N-acylierten Derivaten, insbesondere N-Palmitoyl-Lys-Thr-Thr-Lys-Ser, Val-Gly-Val-Ala-Pro-Gly und seinen N-acylierten Derivaten, insbesondere N-Palmitoyl-Val-Gly-Val-Ala-Pro-Gly, Acetyl-Hexapeptide-3, Hexapeptide-4, Hexapeptide-5, Myristoyl Hexapeptide-5, Myristoyl Hexapeptide-6, Hexapeptide-8, Hexapeptide-9, Hexapeptide-10, L-Glutamylaminoethyl-indol, sowie Kombinationen dieser Substanzen, insbesondere Kombinationen aus N-Palmitoyl-Gly-His-Lys und N-Palmitoyl-Gly-Gln-Pro-Arg.

11. Zusammensetzung nach einem der Ansprüche 1 bis 10, **dadurch gekennzeichnet, dass** die Polymeren von Aminosäuren, N-C₂-C₂₄-Acylaminosäuren und/oder den physiologisch verträglichen Metallsalzen dieser Substanzen ausgewählt sind aus
- tierischen und pflanzlichen Proteinhydrolysaten,
- tierischen und pflanzlichen Proteinen und
- DNA-Reparaturenzymen.

12. Zusammensetzung nach einem der Ansprüche 1 bis 11, **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Früchten von Xanthium sibiricum enthält.

13. Zusammensetzung nach einem der Ansprüche 1 bis 12, **dadurch gekennzeichnet, dass** sie - bezogen auf ihr Gewicht - 0,001 bis 10 Gew.-%, vorzugsweise 0,002 bis 9 Gew.-%, weiter bevorzugt 0,005 bis 8 Gew.-%, noch weiter bevorzugt 0,01 bis 7 Gew.-% und insbesondere 0,05 bis 6 Gew.-% Extrakt(e) aus den Wurzeln von Cyperus rotundus enthält.

14. Zusammensetzung nach einem der Ansprüche 1 bis 13, **dadurch gekennzeichnet, dass** sie - bezogen auf ihr Gewicht - 0,001 bis 10 Gew.-%, vorzugsweise 0,005 bis 7,5 Gew.-%, weiter bevorzugt 0,01 bis 5 Gew.-% und insbesondere 0,02 bis 1 Gew.-% Xanthophylle, vorzugsweise Canthaxanthin, Citranaxanthin, Flavoxanthin, Rhodoxanthin, Rubixanthin, Zeaxanthin und ganz besonders bevorzugt Astaxanthin, enthält.

15. Zusammensetzung nach einem der Ansprüche 1 bis 14, **dadurch gekennzeichnet, dass** sie zusätzlich mindestens ein DNA-Reparaturenzym, vorzugsweise Photolyase und/oder T4 Endonuclease V und/oder 8-Oxoguanin-glycosylase und/oder deren Mischungen enthält.
